# EUROPEAN PATENT APPLICATION

(11) **EP 1 232 764 A1**
(43) Date of publication of application: **21.08.2002**
(21) Application number: 00977868.9
(22) Date of filing: 22.11.2000
(51) Int. Cl.: A61M 25/00

(54) **GUIDE TUBE AND APPLICATION METHOD THEREFOR**

(30) Priority: 25.11.1999 JP 33515499
(71) Applicant: SUMITOMO BAKELITE CO., LTD., Tokyo 140-0002 (JP)
(72) Inventor: Yamamoto, Manabu, Tokyo 120-0005 (JP); Ishi, Kimio, Tokyo 133-0056 (JP); Isono, Tomohiro, Akita Sumitomo Bakelite Co., Ltd., Akita-shi, Akita 011-0951 (JP); Asai, Hideaki, Akita Sumitomo Bakelite Co., Ltd., Akita-shi, Akita 011-0951 (JP); Masuda, Haruhiko, Akita Sumitomo Bakelite Co. Ltd., Akita-shi, Akita 011-0951 (JP)
(74) Representative: Grund, Martin, Dr.
(86) International application number: JP0008244
(87) International publication number: WO01037917

(57) **Abstract**

A guide tube and method for inserting a medical treatment tube with the guide tube, in which the guide tube is drawn toward a mouth side after being inserted into and fixed to a digestive tract, so that the digestive tract is straightened to facilitate the insertion of the medical treatment tube, are provided. The guide tube for inserting the medical treatment tube into a body vessel, has an inner cavity extending through the full length of the guide tube, a helical-shaped or mesh-shaped reinforcing member in a wall forming the guide tube, and, at the vicinity of a distal end thereof, means for fixing guide tube into the digestive tract, to be fixed to the digestive tract while keeping the inner cavity and to straighten the digestive tract by pulling the guide tube toward the mouth side, so that the medical treatment tube can be inserted into the target body vessel.

## Description

### TECHNICAL FIELD

The present invention relates to a guide tube for introducing a catheter, vessel expander or inspection equipment for medical treatment into a body vessel.

### BACKGROUND ART

As examples of the catheter, vessel expander and inspection equipment to be introduced into the body vessel, there is a medical treatment tube such as an ileus tube for diagnosis and medical-treatment of ileus, an enteral nutrition tube or the like, or for releasing an intestinal tract stenosis at small intestine or duodenum, a small intestine endoscope for inspecting the small intestine, and so forth. (Hereafter, these are called as medical treatment tubes.)

However, it is necessary for introducing these medical treatment tubes from mouth or nose into a digestive tract distal than stomach that they pass a portion curved by approximately 180 degrees from cardia along stomach curvature to duodenum pylorus, so that it is difficult that the medical treatment is moved inward by only pushing it, therefore, the medical treatment tube insertion is brought about by directing a front end of the medical treatment tube with attaching a weight to the front end of the medical treatment tube, or repeating a reciprocation of a guide wire inserted into an interior cavity of the medical treatment tube as a stylet, a patient body attitude change, and a reciprocation of the medical treatment tube while performing an X-ray fluoroscopy.

When the medical treatment tube is moved forward in the digestive tract through the pylorus, the weight at the front end of the medical treatment tube or the guide wire is used and the patient body attitude is changed while pushing the medical treatment tube gradually, however, because of the portion curved by approximately 180 degrees from cardia to duodenum pylorus, the pushing force is not effectively transmitted to the front end of the medical treatment tube, therefore, the insertion is impossible for some patients with necessity of a certain time period and technique, and there are problems of patient's pain in nose and mouth and of a long time period exposure of X-ray.

### DISCLOSURE OF THE INVENTION

An object of the present invention is to solve these problems, and a guide tube to be fixed to the digestive tract and to be pulled to make a curved route from the mouth to the pylorus straight when the medical treatment tube is introduced in the body vessel to the pylorus and a method for using it are provided.

That is, the present invention provides,
(1) a guide tube for introducing a catheter, vessel expander or inspection equipment into a body vessel, comprising an inner cavity extending through the whole length of the guide tube, characterized in that a wall forming the tube includes therein a reinforcing member to maintain a space of the inner cavity with preventing the guide tube from being closed when the guide tube is bent to be inserted into the body vessel, so that the catheter, vessel expander or inspection equipment is inserted into a target portion of the body vessel through the inner cavity.
(2) In the guide tube described in (1), the guide tube has, at the vicinity of a distal end thereof, at least one means for fixing the guide tube to a digestive tract.
(3) In the guide tube described in (1), the guide tube has, at the vicinity of a distal end thereof, a balloon to be expanded by an injection of a fluid when being inserted in the body vessel.
(4) In the guide tube described in any one of (1)-(3), the reinforcing member is metallic wires or plastic fiber.
(5) In the guide tube described in (4), an outer diameter of the metallic wire or plastic fiber is 0.1-0.5 mm, and/or a distance between the metallic wires or plastic fiber adjacent to each other is not less than 0.4 mm.
(6) In the guide tube described in any one of (1)-(5), the guide tube has the reinforcing member in the wall over a length not more than two thirds of the whole length of the guide tube from a proximate end of the guide tube.
(7) In the guide tube described in any one of (1)-(6), the body vessel is a digestive tract, and the guide tube has, in the vicinity of a proximate end of the guide tube, a mechanism for fixing the guide tube to a patient's mouth with a mouth piece after the guide tube is inserted.
(8) In the guide tube described in (7), the digestive tract is distal from a stomach.
(9) In the guide tube described in (7), the mechanism is detachably attached to the mouth piece.
(10) In the guide tube described in any one of (1)-(9), a distal end of the guide tube is thinner than the other tube part thereof, and has an obliquely-cut shape.
(11) In the guide tube described in any one of (1)-(10), a part or the whole of a surface of at least one of inner and outer sides of the guide tube is a surface treated for lubrication.
(12) In the guide tube described in any one of (1)-(11), the guide tube has a rib-shaped longitudinally extending protrusion on at least a part of an inner surface thereof.
(13) The present invention provides, a method for using the guide tube described in any one of (1)-(12), characterized by fixing the guide tube into the digestive tract by the fixing means attached to the vicinity of the distal end after inserting the guide tube into the target digestive tract, and subsequently inserting the catheter, vessel expander or inspection equipment into the target digestive tract while holding and pulling the proximate end thereof to straighten the curved digestive tract.

### BRIEF EXPLANATION OF THE DRAWINGS

Fig. 1 is a side view showing a structure of a guide tube of an embodiment of the present invention.

Fig. 2 is a cross sectional view showing a cut surface of the guide tube of the embodiment of the present invention.

Fig. 3 includes (a) part for helical shape structure of metallic wires or plastic fiber of an embodiment of the present invention, and (b) part for mesh-shape structure thereof.

Fig. 4 is a view showing a method for inserting a medical treatment tube into a digestive tract with the guide tube of the present invention.

Fig. 5 is a view showing a method for inserting the medical treatment tube into the digestive tract with the guide tube of the present invention.

Fig. 6 is a view showing a method for inserting the medical treatment tube into the digestive tract with the guide tube of the present invention.

Fig. 7 is a view showing a method for inserting the medical treatment tube into the digestive tract with the guide tube of the present invention.

### EMBODIMENTS OF THE INVENTION

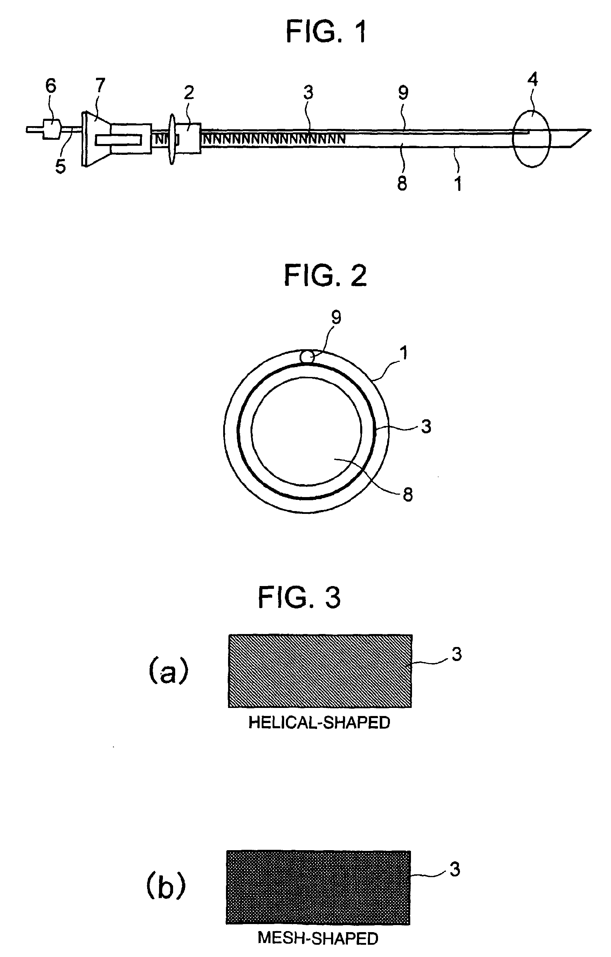

The present invention will be explained in detail hereafter on the drawings. Fig. 1 is a side view showing a structure of a guide tube of an embodiment of the present invention, Fig. 2 is a cross sectional view showing a cut surface of the guide tube of the embodiment of the present invention, and Fig. 3 includes (a) and (b) parts for helical shape structure or mesh-shape structure of metallic wires or plastic fiber.

As an embodiment of the guide tube according to the present invention, in Fig. 1, means for fixing the guide tube into a digestive tract is balloon. A guide tube of the present invention has a guide tube body 1 including a main-lumen 8 through which a medical treatment tube is inserted and a sub-lumen 9 through which a fluid is injected into and discharged from a balloon 4 as the means for fixing the guide tube into the digestive tract, a mouth piece 2 for fixing the guide tube to a patient's mouth, a helical-shaped or mesh-shaped reinforcing member 3 formed by metallic wires or plastic fiber in a wall of the guide tube body 1, the balloon 4 as the means for fixing the guide tube into the digestive tract, a combination of branch tube 5 and one-way-valve syringe connector 6 connected to the sub-lumen 9 so that the fluid is injected into and discharged from the balloon 4, and a cap 7 for fixing the mouth piece 2 in the vicinity of a proximate end of the guide tube to the tube.

The full length of the guide tube body 1, for example, needs to be sufficient for extending at least from the mouth to a pylorus ring as an outlet of the stomach, and is preferably not less than 50 cm, more particularly 50-100 cm with taking a length of the medical treatment tube and operability thereof into consideration. An inner diameter of the guide tube body 1 is at least more than an outer diameter of the medical treatment tube to be used, for example, by 1 % of the outer diameter of the medical treatment tube, for sliding between the guide tube and the medical treatment tube.

An outer diameter of the guide tube body 1 is determined from the inner diameter of the guide tube body 1 and a thickness of the guide tube body 1, and is preferably not more than 25 mm, because if it exceeds 25 mm, the insertion into the digestive tract is very difficult and a pain of the patient increases. As a material of the guide tube body 1, polyvinylchloride resin, polyeurethane resin, polyolefine-type elastomer, styrene-type elastomer, silicone rubber, fluorocarbon-polyethylene resin or a combination of these is usable.

A front end of distal side of the guide tube body 1 has preferably an obliquely-cut shape, particularly a shape cut with an angle of 20-70 degrees effective for decreasing a resistance against the insertion into the digestive tract and preventing a mucous membrane of the digestive tract from being caught into the tube during the insertion. Further, an edge of the cut portion is preferably rounded to prevent the patient's inside body from being injured. The outer diameter of the distal end of the guide tube body 1 smaller in comparison with the adjacent portion is tapered in a range of 10-100m from the front end of the guide tube so that the resistance against the insertion into the digestive tract is decreased.

The helical-shaped or mesh-shaped reinforcing member 3 in the wall of the guide tube is formed by metallic wires or plastic fiber. Further, the reinforcing member 3 is effective for making the outer diameter of the whole of the guide tube as small as possible, preventing, with a sufficient strength, the inner cavity of the tube from being closed when the guide tube is bent in the digestive tract, and holding securely, with a hand, the medical treatment tube inserted from an outside of the guide tube into the inner cavity of the guide tube.

The reinforcing member 3 is preferably formed by stainless steel wire, nickel-titanium alloy wire, polyamide fiber, polypropylene fiber or the like, and a diameter thereof is preferably, 0.1-0.5 mm. And/or, a distance of is at least not less than 0.4 mm, preferably 1.5-5 mm. An angle between the metallic wires or synthetic resin fiber crossing each other is preferably 40-140 degrees.

The guide tube including the reinforcing member has preferably a characteristic for preventing the inner cavity of the tube from being closed when the guide tube is bent in the digestive tract and a characteristic for holding securely, with the hand, the medical treatment tube inserted from the outside of the guide tube into the inner cavity of the guide tube, and a resistance force of 1-15 kgf, preferably 1-10 kgf, more preferably 1-3 kgf, when a length of guide tube is 3 cm, and is compressed by 5 mm in a radial direction of the main-lumen.

The reinforcing member 3 may be arranged over the full length of the guide tube, but the reinforcing member 3 causes a difficulty of insertion into the digestive tract and an injury of the digestive tract because the reinforcing member 3 makes the guide tube hard. Since the curvature of the digestive tract can be straightened by pulling the proximate end of the guide tube toward the mouth side, it is preferable that the reinforcing member is arranged in a portion which is bent most in the digestive tract and is prevented from being arranged in the front end of the guide tube to prevent the injury of the digestive tract and to not restrain a movement of the medical treatment tube, that is, the reinforcing member is arranged in a longitudinal length not more than two thirds of the full length thereof from the proximate end of the guide tube.

A hardness of the whole of the guide tube is very important for preventing the insertion of the guide tube into the digestive tract and the operation of the medical treatment tube from being deteriorated, and a ratio of non-reinforced portion to reinforced portion in resistance force when the length of guide tube to be compressed by 5 mm in the radial direction of the main-lumen is 3 cm is 1 : 1.3 - 1 : 18, preferably 1 : 1.3 - 1 : 2.

The means for fixing the guide tube into the digestive tract may be the balloon, mallecot or the like. Since the digestive tract to which the guide tube is fixed is soft to be easily damaged, it is preferable for the means for fixing the guide tube to be as soft as possible and to be capable of fixing securely, therefore, the balloon is particularly preferable. The means for fixing the guide tube has a shape capable of fixing securely the guide tube body 1 to the pylorus ring as outlet of stomach.

A concrete shape therefor when the means for fixing is expanded, is a ball shape, a conical shape in which an outer diameter increases gradually from the distal end toward the proximate end in the guide tube, or an elliptical shape whose cross sectional shape taken along a longitudinal direction of the guide tube has a minor axis directed to the longitudinal direction of the guide tube and a major axis directed to a radial direction of the guide tube. It is preferable for the shape to be easily inserted into the pylorus ring and to be hardly withdrawn from the pylorus ring.

For example, it is preferable for maximum diameter in the guide tube radial direction and maximum width in the guide tube longitudinal direction of the means for fixing when the means for fixing is expanded, to be 30-150 mm. For example, it is preferable for the means for fixing the guide tube to be arranged within a range of 0-50 cm from the distal end of the guide tube.

The means for fixing the guide tube may be formed by, when the means for fixing is the balloon 4, polyurethane elastomer, plasticized polyvinyl chloride resin, silicone rubber, polyamide elastomer, polyethylene resin, isoprene rubber, crude rubber, polyolefine type rubber, or a combination of these, and an X-ray impermeable material may be additionally included by the means for fixing the guide tube. The X-ray impermeable material, for example, bismuth oxide, bismuth carbonate, barium sulfate or the like, is effective for easily detecting a position of the means for fixing the guide tube.

If it is difficult for the X-ray impermeable material to be included by the means for fixing the guide tube, the X-ray impermeable material or a resin mixed with the X-ray impermeable material may be attached to each of front and back sides of the means for fixing the guide tube. The branch tube 5 connected to the sub-lumen 9 of the guide tube has a strength sufficient for bearing a pressure for injecting the fluid to expand the balloon 4, and may be formed by polyvinylchloride resin, silicone resin, polyurethane resin, polyamide resin, polyethylene resin, fluorocarbon-polyethylene resin or the like.

The cap 7 for fixing the mouth piece 2 to the guide tube has a nail capable of engaging with a hole of the mouth piece 2 to be fixed thereto, is fixed to the vicinity of the proximate end of the guide tube body 1, and is formed by a material capable of adhering easily to the guide tube and of withstanding a plurality of repeated fittings with the mouth piece 2, for example, polyvinylchloride resin, polypropylene resin, polyacetal resin, ABS resin or the like.

The mouth piece 2 is formed by a material capable of withstanding a plurality of repeated fittings with the cap 7 for fixing the mouth piece 2 to the guide tube, for example, polyvinylchloride resin, polypropylene resin, polyacetal resin, ABS resin or the like.

The lubrication treatment may be performed on at least a part of at least one of outer and inner surfaces of the guide tube to improve a sliding condition for the medical treatment tube, and a lubricant material used in the treatment may be hydro-gel, particularly collagen, polyvinyl-pyrrolidone, polyacrylamide or the like are preferable when taking a toxicity for human body into consideration.

The lubricant material is fixed to the guide tube, by bridging the hydro-gel with glutaric aldehyde after coating the guide tube with a liquid solution of the hydro-gel, by bridging a monomer of the hydro-gel with a polymerization agent after coating with the monomer of the hydro-gel, or by fixing with a light irradiation a liquid solution of the hydro-gel denaturalized by a bridging agent such as a photosensitive bridging agent after coating the guide tube with the liquid solution.

The inner side of the guide tube may have longitudinally extending ribs for decreasing a contact area between the medical treatment tube and the guide tube and preventing the inner cavity from being closed when the guide tube is bent, so that a sliding between the medical treatment tube and the guide tube is facilitated.

The ribs on the inner side of the guide tube can be formed integrally with the guide tube through extrusion process, and has, for example, a height and maximum width of 0.5-10 mm, for maintaining a sufficient clearance between the guide tube and the outer diameter of the medical treatment tube. A length of the ribs is preferably equal to the full length of the guide tube, but may be prevented from being arranged at the vicinity of the front end of the guide tube distal side to be softened.

It is preferable for a cross sectional shape of the ribs to be determined in such a manner that the contact area between the medical treatment tube and the guide is as small as possible, and a substantially triangular cross sectional shape which is taped from the wall of the inner cavity of the guide tube toward a center of the inner cavity is especially preferable.

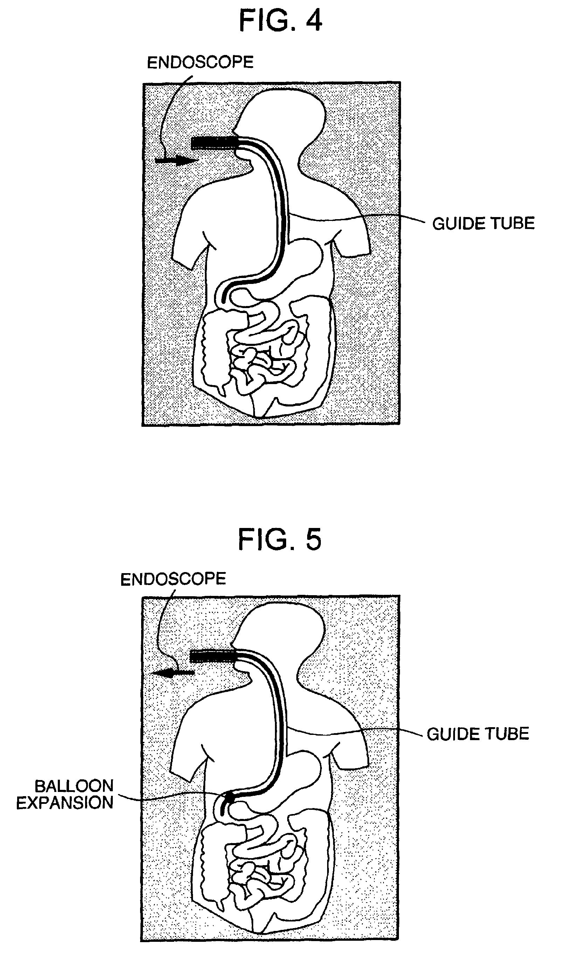

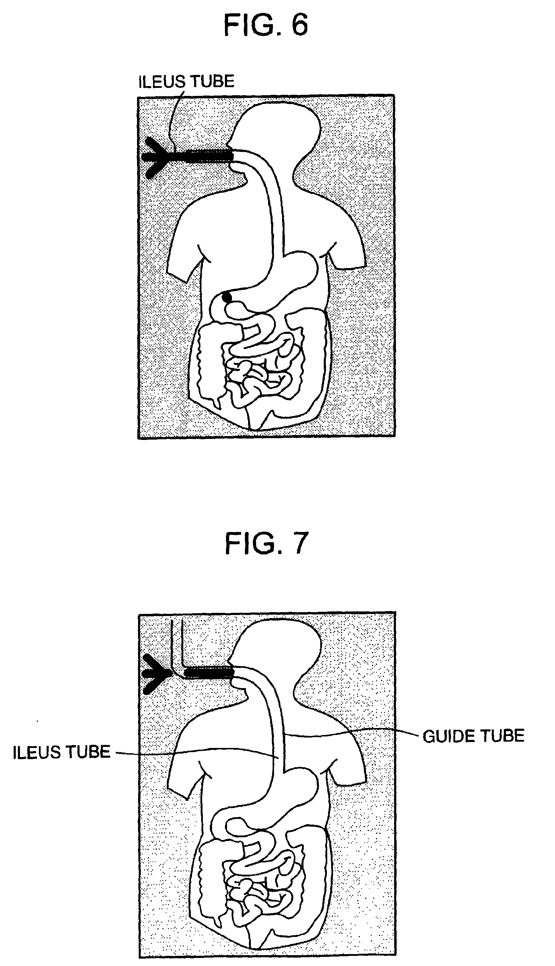

As shown in Fig. 4, in a method for using the guide tube of the present invention, an endoscope is attached in the inner cavity of the guide tube, and the front end of the guide tube is inserted from the mouth to overreach pylorus ball-shaped section of intestine duodenum together with the endoscope. Subsequently, under X-ray radioscopy, the endoscope and the guide tube are operated to position the balloon of the guide tube distal side at the pylorus ball-shaped section. Subsequently, the balloon is expanded to fix the guide tube (Fig. 5). Subsequently, the guide tube is pulled toward the mouth to straighten the digestive tract between the pylorus ball-shaped section and the mouth. Thereafter, the endoscope is withdrawn from the guide tube (Fig. 5), the medical treatment tube is inserted into the straightened inner cavity of the guide tube (Fig. 6), the medical treatment tube is pushed to the target digestive tract, and finally the balloon is contracted to remove the guide tube from the digestive tract (Fig. 7).

As described above, by using the guide tube of the present invention, a route from the mouth to the pylorus is kept straight while maintaining the space of the inner cavity of the tube, the insertion of the medical treatment tube into the pylorus is facilitated, and a time period for the insertion of the medical treatment tube is significantly decreased, so that X-ray exposure on the patient is decreased. Further, since a medical treatment tube in a cavity of nose needs not to be moved when the medical treatment tube is inserted into the digestive tract, a damage of membrana mucosa in the nose cavity is minimized and a pain in the patient's nose cavity is decreased.

## Claims

1. A guide tube for introducing a catheter, vessel expander or inspection equipment into a body vessel, comprising an inner cavity extending through the full length of the guide tube, **characterized in that** a wall forming the tube includes therein a reinforcing member to maintain a space of the inner cavity with preventing the guide tube from being closed when the guide tube is bent to be inserted into the body vessel, so that the catheter, vessel expander or inspection equipment is inserted into a target portion of the body vessel through the inner cavity.

2. A guide tube according to claim 1, wherein the guide tube has, at the vicinity of a distal end thereof, at least one means for fixing the guide tube to a digestive tract.

3. A guide tube according to claim 1, wherein the guide tube has, at the vicinity of a distal end thereof, a balloon to be expanded by an injection of a fluid when being inserted in the body vessel.

4. A guide tube according to any one of claims 1-3, wherein the reinforcing member is metallic wires or plastic fiber.

5. A guide tube according to claim 4, wherein an outer diameter of the metallic wire or plastic fiber is 0.1-0.5 mm, and/or a distance between the metallic wires or plastic fiber adjacent to each other is not less than 0.4 mm.

6. A guide tube according to any one of claims 1-5, wherein the guide tube has the reinforcing member in the wall over a length not more than two thirds of the full length of the guide tube from a proximate end of the guide tube.

7. A guide tube according to any one of claims 1-6, wherein the body vessel is a digestive tract, and the guide tube has, in the vicinity of a proximate end of the guide tube, a mechanism for fixing the guide tube to a patient's mouth with a mouth piece after the guide tube is inserted.

8. A guide tube according to claim 7, wherein the digestive tract is distal from a stomach.

9. A guide tube according to claim 7, wherein the mechanism is detachably attached to the mouth piece.

10. A guide tube according to any one of claims 1-9, wherein a distal end of the guide tube is thinner than the other tube part thereof, and has an obliquely-cut shape.

11. A guide tube according to any one of claims 1-10, wherein a part or the whole of a surface of at least one of inner and outer sides of the guide tube is a surface treated for lubrication.

12. A guide tube according to any one of claims 1-11, wherein the guide tube has a rib-shaped longitudinally extending protrusion on at least a part of an inner surface thereof.

13. A method for using the guide tube according to any one of claims 1-12, **characterized by** fixing the guide tube into the digestive tract by the fixing means attached to the vicinity of the distal end after inserting the guide tube into the target digestive tract, and subsequently inserting the catheter, vessel expander or inspection equipment into the target digestive tract while holding and pulling the proximate end thereof to straighten the curved digestive tract.
